# EUROPEAN PATENT APPLICATION

(11) **EP 2 918 241 A1**
(43) Date of publication of application: **16.09.2015**
(21) Application number: 12888063.0
(22) Date of filing: 08.11.2012
(51) Int. Cl.: A61C 13/09, A61C 13/00, A61K 6/083

(54) **COMPOUND REINFORCED WITH QUARTZ OR GLASS FIBRE AND FLUID PHOTO-CURING RESIN, METHOD FOR RECONSTRUCTING TEETH AND METHOD FOR USING SAID COMPOUND**

(71) Applicant: Rodríguez Posada, Mario Alberto, San José (CR)
(72) Inventor: Rodríguez Posada, Mario Alberto, San José (CR)
(74) Representative: Carvajal y Urquijo, Isabel
(86) International application number: PCT/CR2012/000004
(87) International publication number: WO 2014/071893

(57) **Abstract**

The present invention relates to a compound for medical and dental use made from glass or quartz fiber and light curing liquid resin; the design of dental structures in the mouth and outside the mouth using said compound; and the presentation of the compound as a treatment method.

## Description

### BACKGROUND OF THE INVENTION

The present invention relates to a compound that serves as a medical device for dental use and the process for the reconstruction of teeth in the mouth as well as the treatment method for the application of the compound.

The comprehensive treatment of full mouth rehabilitation consists of the restoration of the dental and periodontal tissues affected, which can be carried out through different dental procedures, ensuring satisfactory oral health for the patient.

Currently, the preferred method for the rehabilitation of lost teeth is with titanium dental implants. These implants replace the roots of the original tooth that is to be restored. It takes three to eight months to achieve the Osseo integration of the dental implant, at which time you may set crowns, dentures or fixed bridges. With this procedure it is not necessary to wear down adjacent teeth, rendering it a conservative, aesthetic and functional type of treatment.

In summary, we can state that the following scientific alternatives are available for the restoration of missing teeth:
- Porcelain /metal fixed dental prostheses
- Zirconia based fixed dental prostheses
- Injected ceramic fixed dental prostheses
- Removable acrylic prostheses
- Removable cobalt-chromium prostheses
- Dental implantsFixed dental rehabilitation can be carried out using metal-porcelain, zirconium or injected ceramic. These treatments require excessive wearing down of the teeth adjacent to the edentulous space that is to be restored.

While patents exist that serve as precursors for the prior art, their properties differ from our invention. For example, existing innovations to prior art include prostheses (for example, WO2005120438) or the construction of parts outside of the mouth.

Other innovations include materials made of fiberglass and resin (for example, KR20020095396 and EP0973481), but none include the mixture of the components of the present invention, which results in a product that can be manipulated within the mouth. Another invention presents the incorporation of fiber to the acrylic powder used, to make dentures that are stronger (for example, EP0913130). Most existing techniques allow for the reconstruction of pieces in the lab but none allow for work to be carried out in the mouth, resolving a series of complex clinical problems. At best, the prior art includes inventions that address the clinical problem of the space left by one absent tooth, but not for the absence of several pieces (for example, EP0610187, WO2004100816, RU02286113 and RU02320292).

While invention WO1993008760 establishes that the use of fiberglass in dental reconstruction is not new, the fiber that is currently in use is not easily manipulated and, in that sense, differs from the materials used in the present invention. In the case of patent EP1628592, while it does use a similar technique, in that it also refers to dental restoration through a compound reinforced with one or more fiber structures in different ways, done in incremental layers, it differs from the present invention because the components are different, which allows the present invention to be a compound that is much more resistant, easier to manipulate and can be worked on while in the mouth.

Innovations to prior art using fiber include well-known techniques with common fiber (random fiber) that have been used for years now for the rehabilitation of spaces left by the extraction of teeth (for example, US6200136) differ from the present invention, resulting in a compound that allows for a qualitative improvement in strength and duration, that is also much easier to manipulate.

### DETAILED DESCRIPTION OF THE INVENTION

Several years of clinical trials with patients using the dental restoration compound that is the object of the present invention have allowed us to perfect the compound, its technique and consistently test its efficacy in different treatments.

A variety of techniques for dental rehabilitation, reconstruction and aesthetic treatments found around the world make up the known art. The present invention is a conservative, aesthetic and functional technique because the physical properties of the material addresses clinical situations that would otherwise have to resort to other fixed restoration alternatives involving the excessive wear of adjacent teeth, such as fixed bridges, crowns, periodontal splints, increased vertical dimension.

### ABOUT THE COMPOUND

The object of this invention is a compound made up of glass or quartz fiber mixed with a bis-GMA (bisphenol glycidyl methacrylate) based light curing resin compound with little inorganic filler, known as fluid resin.

The glass or quartz fiber is cut into linear fragments that are 3 to 20 millimeters in length and 0.5 to 2 millimeters in circumference. The fragments are placed on adhesive tape to facilitate handling and storage.

Fluid resin is gradually added to the glass or quartz fiber fragments until a thick, dense, uniform mass, that is easy to mold, is obtained.

The tape with the resulting compound must then be placed in a storage container to avoid exposure to light, to maintain its consistency and to prevent photo-polymerization.

### ABOUT THE PROCESS FOR THE RECONSTRUCTION OF TEETH

The compound described can be used for the construction of immediate fixed bridges, for the reconstruction of damaged teeth, for periodontal splints, stumps and increased vertical dimension, all directly within the mouth, as well as for crowns and fixed bridges that can be made outside the mouth.

The process for the reconstruction of teeth using the compound described consists of the following series of steps:

Identify the area that is to be rehabilitated in the mouth, prepare the tooth or neighboring teeth by wearing as needed, based on each clinical case, and isolate the clinical field. Once the isolated clinical field has been etched with 35% phosphoric acid for 10 seconds for dentin and 15 seconds for enamel, rinse the area with abundant water. Dry the clinical areas, while striving to maintain some moisture, and apply the light-curing total etch adhesive, or any other similar adhesive. Apply air to remove any excess adhesive. Light cure the field for 20 to 40 seconds and measure the distance between teeth (interdental space) to determine the size of the compound fragments needed to begin closing the space between the teeth.

The compound is applied in fragments that are light cured individually, until a mass is formed in the mouth. Fluid resin is applied between each fragment. Light curing hardens the mass. The compound fragments are added vertically, horizontally or sideways, depending on the area that needs to be filled to shape the teeth.

Once the compound based structure is complete, shape the structure using bits. Adjust as needed to leave room for the last layer of the bis-GMA based light curing resin compound. Apply 35% phosphoric acid for 10 seconds and then rinse with abundant water. Apply the total etch adhesive, or a similar adhesive, and light cure for 40 seconds, if necessary, one or two times. Apply the light curing resin compound incrementally and light cure each layer. It is important to cover the fiber-based structure with the resin compound that will later be shaped using diamond and carbide drill bits. Last, polish the structure using traditional methods.

### ABOUT THE TREATMENT METHOD

The compound can be used for the reconstruction of teeth that are very damaged, for the construction of periodontal splints, immediate fixed bridges, stumps undergoing endodontic treatment, extremely damaged vital teeth, increased vertical dimension, all within the mouth, as well as crowns and fixed bridges outside of the mouth.

## Claims

1. A compound of glass or quartz fiber and a bis-GMA (bisphenol glycidyl methacrylate) based light curing resin compound, known as fluid resin, wherein:
fluid resin is added to the glass or quartz fiber fragments until they are saturated, producing a uniformly dense, thick mass that can be shaped.

2. A compound of claim 1, wherein the glass or quartz fiber is cut into linear fragments of 3 to 20 millimeters in length and 0.5 to 2 millimeters in circumference.

3. A compound of claim 1, wherein the fluid resin is a bis-GMA (bisphenol glycidyl methacrylate) based light curing resin compound with little inorganic filler.

4. A process to design dental structures within the mouth with the compound from claim 1, which includes the following steps:
Select the space that is to be restored between 1 to 4 neighboring teeth.
In the mouth, prepare the teeth with retention walls, with the minimum amount of wear, based on the clinical case;
Isolate the clinical field;
Etch with 35% phosphoric acid for 10 seconds for dentin and 15 seconds for porcelain;
Rinse the area with abundant water;
Dry the clinical field while striving to maintain some moisture;
Apply the light curing total etch adhesive, or a similar adhesive, to the clinical field;
Apply air to remove any excess adhesive;
Light cure the field for 20 to 40 seconds;
Measure the distance between teeth;
Apply a very thin layer of fluid resin to the cavity but do not light cure;
In the mouth, apply the compou7nd from claim 1 to the distance between teeth in layers, until the space is filled and there is a good fit in the space;
A base of fluid resin is applied for each application of the compound;
Each application of the fluid resin compound is light cured for 20 to 40 seconds;
Compound fragments from claim 1 are incorporated vertically,
horizontally or sideways, depending the space to be filled to mold the teeth;
Once the compound based structure is complete, it is molded using diamond and carbide bits, wearing as to make room for the last layer of the light curing resin compound;
Apply phosphoric acid for 15 seconds;
Rinse with abundant water;
Apply the total etch adhesive, or a similar adhesive and light cure for 20 to 40 seconds;
The result is then coated with the bis-GMA based light curing resin;
The mass is shaped using diamond and carbide drill bits until the shape of the tooth is obtained and proper occlusion is achieved;
Last, polish the structure using traditional methods.

5. A storage and dosing process for the compound of claim 1, wherein:
it comes in a presentation of linear fragments of 3 to 20 millimeters in length and 0.5 to 2 millimeters in circumference;
they are placed on transparent adhesive tape and saturated with fluid resin;
they are stored in a light proof container to avoid photo-polymerization.

6. A compound of claim 1, wherein:
it can be used for immediate bridges in the mouth;
its application depends on neighboring teeth for one to four spacers;
it is applied in fragments that are individually light cured until a mass is formed;
it is saturated in a conventional bis-GMA based light curing resin compound;
it is polished to shape.

7. A compound of claim 1, which can be used for the reconstruction of one single tooth, in the mouth.

8. A compound of claim 1, which can be used for the construction of periodontal splints.

9. A compound of claim 1, which can be used for the construction of fixed bridges, outside of the mouth.

10. A treatment method that consists of dental restoration using the compound of claim 1, in which it can be applied in the construction of stumps, periodontal splints, increased vertical dimension, immediate fixed bridges, the reconstruction of severely damaged teeth (all of which can be carried out in the mouth) as well as crowns and fixed bridges outside of the mouth.
